**Europäisches Patentamt**

**European Patent Office**

**Office européen des brevets**

(11) Veröffentlichungsnummer : **0 516 582 A1**

## (12) EUROPÄISCHE PATENTANMELDUNG

(21) Anmeldenummer : **92810313.4**

(22) Anmeldetag : **29.04.92**

(51) Int. Cl.$^5$ : **A61B 8/08, A61B 17/34**

(30) Priorität : **27.05.91 CH 1561/91**

(43) Veröffentlichungstag der Anmeldung :
**02.12.92 Patentblatt 92/49**

(84) Benannte Vertragsstaaten :
**AT CH DE ES FR GB IT LI NL SE**

(71) Anmelder : **GEBRÜDER SULZER
AKTIENGESELLSCHAFT
Zürcherstrasse 12
CH-8401 Winterthur (CH)**

(72) Erfinder : **Dardel, Eric, Dr. med.
Stationsstrasse 10
CH-8472 Seuzach (CH)**

(54) **Punktiergerät für Blutgefässe.**

(57)    Das Punktiergerät für Blutgefässe weist eine Ultraschallsonde (20) auf, die eine auf dem Dopplereffekt beruhende Ortung des Blutgefässes (50) ermöglicht. Mittels eines Führungskanals (13) lässt sich die Punktiernadel (30) zumindest angenähert koaxial mit dem durch die Sonde (20) emittierten Strahl führen. Das Punktieren muss steril durchgeführt werden. Dazu wird erfindungsgemäss die Sonde mit einer sterilen Hülle ummantelt, wobei diese Hülle ein steriles Koppelstück (1) aufweist, in welchem ein Spiegel (12) für den Ultraschallstrahl und ein Nadelführungskanal (13) angeordnet sind. Das Koppelstück (1) kann ein Teil der Hülle sein ; es kann aber auch als ein auf die Hülle aufsetzbares Zusatzstück vorgesehen werden. Die sterile Hülle mit dem Koppelstück (1), die zum einmaligen Gebrauch bestimmt ist, macht zeitaufwendige Gassterilisationen des Punktiergeräts unnötig.

Fig. 2

Fig. 3

EP 0 516 582 A1

Die Erfindung betrifft Punktiergeräte für Blutgefässe gemäss Oberbegriff von Anspruch 1 sowie sterile Hüllen zu solchen Punktiergeräten. Die Ultraschallsonde eines solchen Geräts weist eine Sender/Empfänger-Anordnung auf, mit der Ultraschall in den Körper eingestrahlt und ein Signal des teilweise reflektierten Strahls registriert werden kann. Durch Reflexion am strömenden Blut ergibt sich dank des Dopplereffekts eine Fequenzverschiebung, aus der sich ein akustisches und/oder optisches Signal gewinnen lässt. Durch Suchen eines Maximums eines solchen Signals kann die Einstichstelle für die Punktiernadel gefunden werden.

Derartige Geräte sind beispielsweise aus der CH-PS 676 787 (= P.6297) bekannt. Die Sender/Empfänger-Anordnung weist eine Konstruktion auf, bei der ein Führungskanal für die Nadel durch den Schallschwinger verläuft. Diese Konstruktion ist nachteilig, da das Punktieren unter sterilen Bedingungen durchgeführt muss und da eine notwendige Gassterilisation der Ultraschallsonde sehr zeitaufwendig ist. Bei einer einfacheren Ultraschallsonde, die keinen integrierten Führungskanal aufweist, lässt sich die Sterilität durch eine sterile, zum einmaligen Gebrauch bestimmte Hülle erreichen (siehe beispielsweise EP-A 0 104 618). Bei diesen einfacheren Sonden kann allerdings die Nadel nicht koaxial zum emittierten Ultraschallstrahl geführt werden.

Es ist Aufgabe der Erfindung, ein Punktiergerät zu schaffen, deren Ultraschallsonde mit einer sterilen Hülle ummantelt werden kann, wobei eine zur Richtung des emittierten Strahls zumindest angenähert koaxiale Nadelführung möglich ist. Diese Aufgabe wird durch die kennzeichnenden Merkmale des Anspruchs 1 gelöst. Ein Koppelstück, das den Ultraschall unter Reflexion an einer Spiegelfläche in den Körper lenkt und in welchem der Nadelführungskanal angeordnet ist, wird aus sterilem oder sterilisierbarem Werkstoff hergestellt. Dieses Koppelstück kann ein Teil der sterilen Hülle sein; es kann aber auch als ein auf die Hülle aufsetzbares Zusatzstück vorgesehen werden. Die sterile Hülle mit dem Koppelstück ist vorzugsweise zu einmaligem Gebrauch bestimmt.

Bei einem kompakten Punktiergerät, das in der Hand gehalten werden kann, bildet man die Hülle solcherart aus, dass das Gerät vollständig umschlossen wird. Bei anderen Punktiergeräten, bei denen beispielsweise die optischen und/oder akustischen Anzeigemittel in einem stationären Geräteteil, das separat aufgestellt wird, untergebracht sind, genügt es, wenn nur das Punktiergerät im Behandlungsbereich (d.h. Sonde und evtl. ein Teil des Verbindungskabels) steril ummantelt wird.

Das Koppelstück kann aus einem festen, Ultraschall gut leitenden Werkstoff gefertigt sein, wobei der Ultraschallspiegel beispielsweise durch eine freie äussere Oberfläche gebildet wird, die der Schalloptik entsprechend angeordnet ist. Das Koppelstück kann auch aus einem Gehäuse bestehen, das mit einem Ultraschall gut leitenden Gel gefüllt ist, wobei der Spiegel beispielsweise aus einem metallischen Körper bestehen kann, der gleichzeitig einen Teil der Gehäusewand sowie den Nadelführungskanal bildet.

Nachfolgend wird die Erfindung anhand verschiedener Ausführungsbeispiele im Zusammenhang mit der Zeichnung näher erläutert. Es zeigen:

Fig. 1 ein erstes Ausführungsbeispiel einer Ultraschallsonde mit einer sterilen Hülle gemäss der Erfindung,

Fig. 2 eine perspektivische Darstellung eines zweiten Ausführungsbeispiels,

Fig. 3 einen Längsschnitt durch das Koppelstück des zweiten Ausführungsbeispiels,

Fig. 4 das Koppelstück eines dritten Ausführungsbeispiels mit einer Kunststoffmembran anstelle eines metallischen Körpers als Ultraschallspiegel,

Fig. 5a nichtsteriles Teil eines kompakten Punktiergeräts,

Fig. 5b den Längsschnitt durch eine erfindungsgemässe Hülle zum Geräteteil der Fig.5a (viertes Ausführungsbeispiel),

Fig. 6a einen Sondenkopf mit aufsetzbarem Koppelstück und

Fig. 6b das Koppelstück der Fig.6a mit einem gelgefüllten "Ballon".

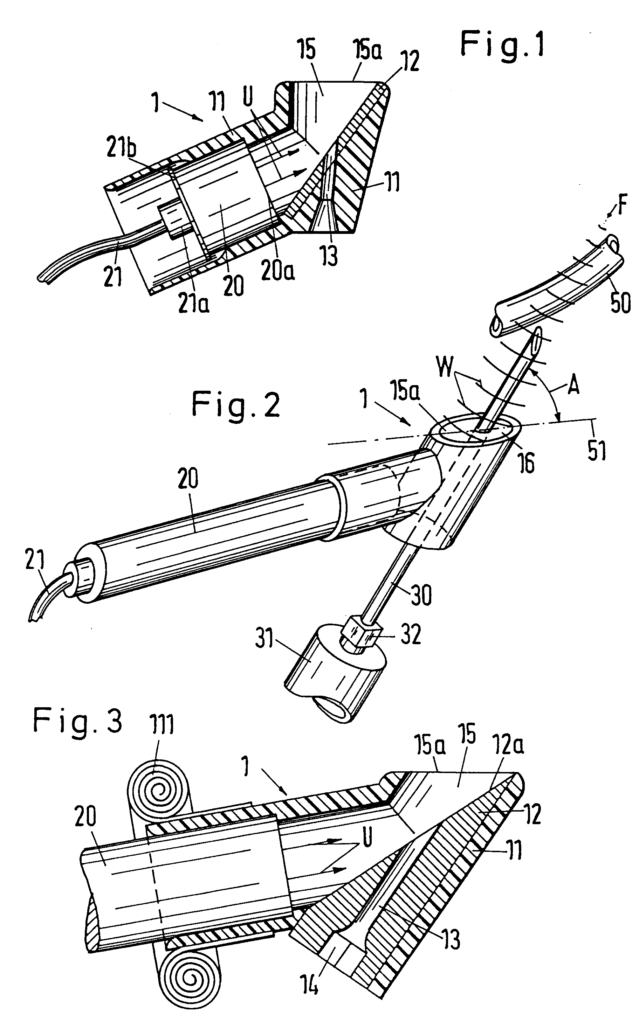

Beim ersten Ausführungsbeispiel in Fig.1 sind folgende Teile zu sehen: Das Koppelstück 1 mit dem Gehäuse 11, das beispielsweise aus Kunststoff spritzgegossen ist, dem Ultraschallspiegel 12 (oder Reflektor für den Ultraschallstrahl), der aus einem ebenen Metallplättchen besteht, und dem Nadelführungskanal 13, der den Spiegel im mittleren Bereich durchquert; ferner die Ultraschallsonde 20, die mit einem nicht dargestellten stationären Teil des Punktiergerätes über das Kabel 21 verbunden ist.

Die Ultraschallsonde 20 emittiert einen Ultraschallstrahl (symbolisiert durch die beiden Pfeile U) in den von einem nicht dargestellten Koppelungsgel gefüllten Hohlraum 15, wobei der Strahl am Spiegel 12 zur Austrittsöffnung 15a reflektiert wird. Die Austrittsöffnung 15a ist im vorliegenden ersten Ausführungsbeispiel kreisförmig, und der Ultraschallstrahl tritt senkrecht zu dieser Öffnung aus. Der Nadelführungskanal 13 ist entsprechend auch senkrecht zur Austrittsöffnung 15a orientiert. Bei der Anwendung des Punktiergeräts wird die Austrittsöffnung 15a geneigt zur Körperoberfläche gehalten und der keilförmige Zwischenraum mit Koppelungsgel gefüllt.

Die Sonde 20 lässt sich in das Gehäuse 11 einschieben. Mit Vorteil wird die Sonde 20 solcherart ausgebildet, dass das Verbindungskabel 21 mit einem Stecker 21a und einer Abdeckplatte 21b auf den Sondenkopf aufgesteckt werden kann. Dieses Verbindungskabel 21 sowie das Koppelstück 1 werden in sterilem Zustand verpackt und erst unmittelbar vor der Behandlung ausgepackt und mit der Ultraschallsonde 20 zu einem gebrauchsbereiten, sterilen Punktiergeräte zusammengesteckt. Dabei muss selbstverständlich durch geeignete Massnahmen - beispielsweise durch Verwendung von sterilen Zangen - dafür gesorgt werden, dass keine Kontaminierung der äussern Oberflächen des Koppelstückes 1 und der Abdeckplatte 21b, welche die sterile Sondenhülle bilden, durch die nichtsterile Sonde 20 entsteht.

Beim Einschieben der Sonde 20 in das Gehäuse 11 kann eine Luftblase zwischen der Sondenstirnfläche 20a und dem Koppelungsgel im Hohlraum 15 eingeschlossen werden. Daher wird mit Vorteil ein Entlüftungskanal, beispielsweise eine Bohrung oder Nut in der Gehäusewand 11 (nicht dargestellt), vorgesehen.

Beim zweiten Ausführungsbeispiel (Fig.2, Fig.3) ist die Ultraschallsonde 20 stabförmig, und das Koppelstück 1 lenkt die Ultraschallwellen (durch Kreisbögen W symbolisiert) unter einem spitzen Winkel A durch die elliptische Austrittsöffnung 15a. Hier kann die Austrittsöffnung 15a bündig auf die Körperoberfläche (durch die strichpunktierte Linie 51 angedeutet) aufgelegt werden. Die Nadel 30, die auf einer Spritze 31 aufgesetzt ist, wird entlang dem konvergenten Ultraschallstrahl (Fokus F) - nach Ortung des Blutgefässes 50 mittels des Dopplereffektes - in dieses Blutgefäss 50 hineingestossen. In Fig.2 ist das Koppelungsgel 16 angedeutet, mit dem der Hohlraum 15 (Fig.3) ausgefüllt ist.

Der Ultraschallspiegel 12 des zweiten Ausführungsbeispiels wird durch einen schräg angeschnittenen Kreiszylinder gebildet, der beispielsweise aus Stahl besteht. Die als Nadelführung dienende Längsbohrung 13 kann zentral oder auch etwas exzentrisch verlaufen. An der Mündung 14 des Kanals 13 ist dessen Querschnitt solcherart erweitert, dass er formschlüssig beispielsweise auf einen Vierkant 32 passt, der am hintern Ende der Nadel 30 vorgesehen ist und der folgenden Verwendungszweck hat: Nach erfolgter Punktierung des Blutgefässes 50 muss die Spritze 31 von der Nadel 30 entfernt werden. Dazu verschiebt man die Sonde 20 mit Koppelstück 1 entlang der festgehaltenen Nadel 30 nach hinten und führt dabei den Vierkant 32 in die Kanalmündung 32 ein. Während die Sonde 20 weiter mit der einen Hand gehalten wird, kann nun mit der andern Hand die Spritze 31 von der Nadel entfernt werden; ein Mitdrehen der Nadel 30 wird dabei dank des Vierkants 32 verhindert. Selbstverständlich können anstelle des Vierkants 32 und der entsprechenden Kanalmündung 14 auch andere Mittel gewählt werden, mittels derer ein Mitdrehen der Nadel 30 blockierbar ist.

Unmittelbar vor dem Punktieren wird das sterile Koppelstück 1 auf die stabförmige Ultraschallsonde aufgesetzt, wobei der durch das Koppelstück nicht abgedeckte Bereich der Sonde zuvor beispielsweise mit einer sterilen Hülse oder einem sterilen Latexschlauch ummantelt worden ist. Ein aufgewickelter Schlauch 111 aus einem elastomeren Material kann auch - wie in Fig.3 dargestellt - auf dem Koppelstück 1 angeordnet sein. Dieses Koppelstück 1 wird auf die nichtsterile Sonde aufgesteckt, wobei anschliessend der Schlauch 111 auf die Sonde abgewickelt werden kann. Mit dem Schlauch 111 lässt sich dabei auch ein Stück des Verbindungskabels 21 umhüllen, das somit in diesem Fall nicht steril zu sein braucht.

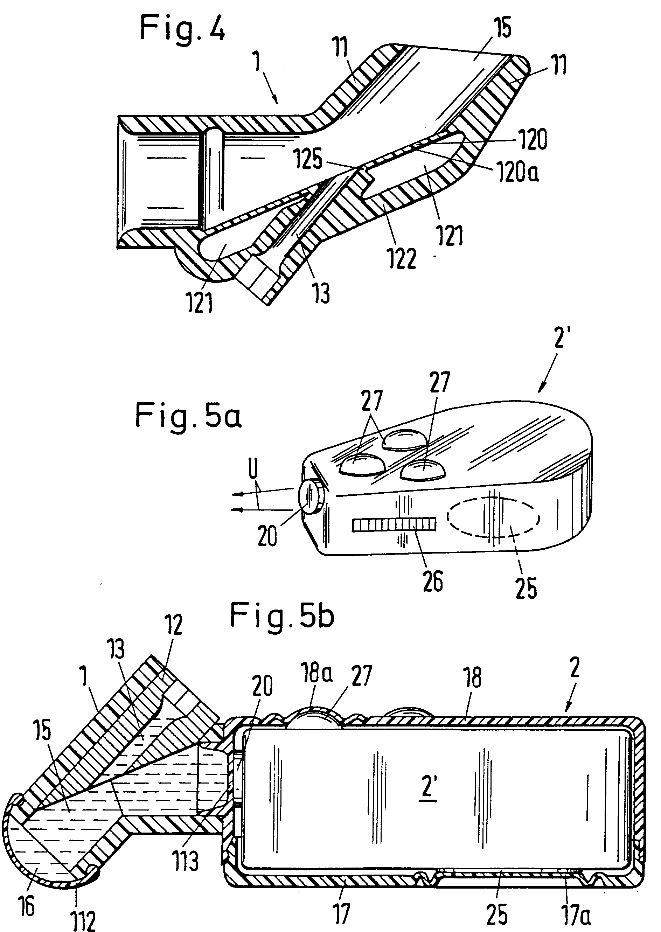

Der Ultraschallspiegel 12 kann aus einem dichten Werkstoff wie beispielsweise Metall oder Glas gefertigt sein. Es geht jedoch auch anders: Fig.4 zeigt eine Ausführungsform, bei welcher der Ultraschallspiegel durch eine Membran 120 gebildet wird, die vor einem Hohlraum 121 aufgespannt ist. Der Ultraschall, der sich im gelgefüllten Hohlraum 15 sowie in der Membran 120 fortpflanzt, wird an der Grenzfläche 120a zwischen der Membran 120 und dem Hohlraum 121 reflektiert. Das Wandstück 122 bildet den Rahmen, über den die Membran 120 gestreckt gehalten wird. An der Durchtrittsstelle des Nadelführungskanals 13 durch die Membran 120 findet keine Reflexion des Ultraschalls statt. Daher wird mit Vorteil in der Nähe der Membran 120 die Kanalwand 125 dünn ausgebildet.

Beim vierten Ausführungsbeispiel (Fig.5a, Fig.5b) handelt es sich um ein kompaktes Punktiergerät 2, das batteriebetrieben ist und das akustische sowie optische Anzeigemittel, beispielsweise einen Lautsprecher 25 und eine Kette von Leuchtdioden 26, aufweist, mit welchen die auf dem Dopplereffekt beruhenden Signale angezeigt werden können. Ferner weist dieses Gerät Bedienungsknöpfe 27 auf, mit denen das Gerät ein- oder ausgeschaltet und beispielsweise die Lautstärke reguliert werden kann. Das Punktiergerät 2 besteht einerseits aus dem nichtsterilen Geräteteil 2' mit der Ultraschallsonde 20, den Anzeigemitteln 25 sowie 26 und den Bedienungsknöpfen 27 und andererseits aus der erfindungsgemässen sterilen Hülle, die sich aus der untern Schale 17 und der obern Schale 18 mit dem Koppelstück 1 zusammensetzt. Die Hülle ist solcherart ausgebildet, dass zwischen Lautsprecher 25 und Hüllenwand 17a eine gut schallabstrahlende Ankoppelung besteht und dass die Hüllenwand 18a bei den Bedienungsknöpfen 27 flexibel ist, sodass diese Knöpfe gedrückt werden können. Damit die optische Anzeige 26 sichtbar ist, muss die Hülle aus einem durchsichtigen Material gefertigt sein.

Das Koppelstück 1 ist beim vierten Ausführungsbeispiel ähnlich ausgebildet wie beim zweiten (Fig.3); selbstverständlich sind hier auch andere Ausführungsformen möglich. In Fig.5b sind weitere Merkmale des Koppelstücks 1 dargestellt, die auch bei den anderen Beispielen realisierbar sind: Das Koppelstück 1 ist körperseitig mit einer Membran 112 aus elastomerem Material abgeschlossen und das Koppelungsgel 16 wird gegen die Sonde 20 durch eine Membran 113 separiert. Durch die konvexe Form der flexiblen Membran 112 lässt sich das Koppelstück 1 anschmiegsam an die Körperoberfläche anlegen. Beim Punktieren wird die Membran 112 mit der Nadel durchstochen. Mit einer durchstechbaren Membran kann auch der Nadelführungskanal 13 verschlossen werden (nicht dargestellt), um ein Austreten von Gel 16 aus dem Hohlraum 15 zu verhindern. Die Membran 113, die sehr dünn sein kann, muss dicht auf der Abstrahlfäche der Ultraschallsonde 20 aufliegen, um Verluste bei der Schallübertragung an dieser Grenzfläche möglichst gering zu halten.

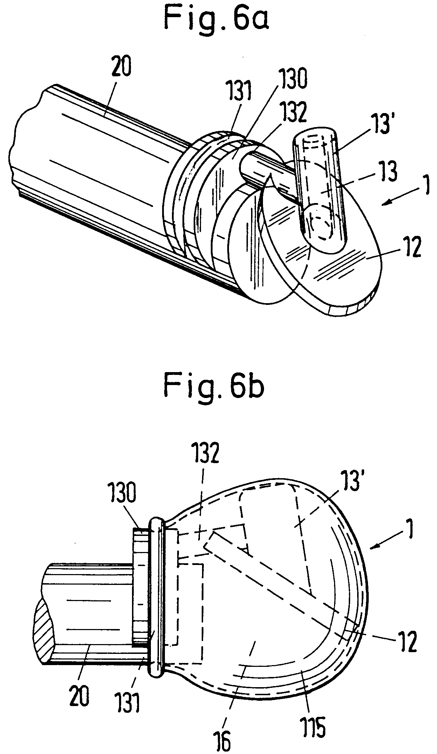

Das Koppelstück 1 des vierten Ausführungsbeispiels kann als Zusatzstück der sterilen Hülle, die aus den beiden Schalenteilen 17 und 18 gebildet wird, angesehen werden. In Fig.6a ist ein anderes Beispiel für ein als Zusatzstück ausgebildetes Koppelstück 1 dargestellt. Dieses Koppelstück 1 wird auf eine Sonde 20 aufgesetzt, die zuvor mit einem sterilen, sackartigen Schlauch aus einem elastomeren Material umhüllt worden ist. Das Koppelstück 1 besteht aus dem Spiegel 12, der Nadelführung 13′ (mit dem Nadelführungskanal 13), der Spange 130, die beispielsweise eine Nut 131 aufweist, und dem Verbindungsstück 132. Der Zwischenraum zwischen Sonde 20, Spiegel 12 und Körperoberfläche muss selbstverständlich wieder mit Koppelungsgel 16 gefüllt sein. Mit einem gelgefüllten und das Koppelstück 1 umhüllenden "Ballon" 115, wie es in Fig.6b dargestellt ist, lässt sich auf praktische Weise diese Forderung erfüllen.

**Patentansprüche**

1. Punktiergerät für Blutgefässe (50), mit einer Ultraschallsonde (20) für auf dem Dopplereffekt beruhenden Ortung des Blutgefässes (50), mit einem Führungskanal (13) für eine Punktiernadel (30), durch den am Eintritt in den Körper die Nadelachse zumindest angenähert koaxial mit dem durch die Sonde (20) emittierten Strahl bewegbar ist,

dadurch gekennzeichnet, dass zwischen Sonde (20) und Körperoberfläche ein steriles Koppelstück (1) angeordnet ist, das den Ultraschallstrahl umlenkt, das den Nadelführungskanal (13) aufweist und das Teil oder Zusatzstück einer Hülle ist, mit der die Sonde (20) zumindest teilweise steril umschliessbar ist.

2. Punktiergerät nach Anspruch 1, dadurch gekennzeichnet, dass das Koppelstück (1) aus einem Gehäuse (11), dessen eine Wand als Spiegel (12) für den Ultraschallstrahl ausgebildet ist, und einem das Gehäuse 11 füllenden Koppelungsgel (16) besteht, wobei im mittleren Bereich des Spiegels (12) der Nadelführungskanal (13) angeordnet ist,

3. Punktiergerät nach Anspruch 2, dadurch gekennzeichnet, dass das Koppelstück (1) körperseitig mit einer Membran (112) aus elastomerem Material abgeschlossen ist.

4. Punktiergerät nach einem der Ansprüche 2 oder 3, dadurch gekennzeichnet, dass das Koppelungsgel (16) gegen die Sonde (20) durch eine Membran (113) abgeschlossen ist.

5. Punktiergerät nach einem der Ansprüche 2 bis 4, dadurch gekennzeichnet, dass zwischen Nadelführungskanal (13) und Koppelungsgel (16) eine Membran angeordnet ist, die mit der Nadel (30) durchstechbar ist.

6. Punktiergerät nach einem der Ansprüche 2 bis 5, dadurch gekennzeichnet, dass der Ultraschallspiegel (12) aus einem dichten Werkstoff wie beispielsweise Metall oder Glas gefertigt ist.

7. Punktiergerät nach einem der Ansprüche 1 bis 6, dadurch gekennzeichnet, dass das Koppelstück (1) auf die Sonde (20) aufsteckbar ist und an der Verbindungsstelle zur Sonde (20) einen aufgewickelten Schlauch (111) aus elastomerem Material aufweist, der über die Sonde (20) abwickelbar ist.

8. Punktiergerät nach einem der Ansprüche 1 bis 6, dadurch gekennzeichnet, dass das Punktiergerät batteriebetrieben ist, akustische und/oder optische Anzeigemittel (25, 26) sowie Betätigungsknöpfe (27) aufweist und in eine mindest zweiteilige Hülle (17, 18) einschliessbar ist, wobei diese Hülle zumindest teilweise schallübertragend, durchsichtig und flexibel ausgebildet ist.

9. Punktiergerät nach einem der Ansprüche 1 bis 6, dadurch gekennzeichnet, dass das Koppelstück (1) auf eine mit einer sterilen Hülle zumindest teilweise ummantelten Sonde (20) aufsetzbar ist.

10. Punktiergerät nach Anspruch 1, dadurch gekennzeichnet, dass das Koppelstück (1) aus einem Ultraschallspiegel (12), einer Nadelführung (13′) und einem Koppelungsgel (16) besteht, wobei diese Komponenten (12, 13′, 16) von einer ballonartigen Membran (115) umschlossen sind.

11. Punktiergerät nach einem der Ansprüche 1 bis 10, dadurch gekennzeichnet, dass am Nadelführungskanal (13) und entsprechend an der Nadel (30) Mittel vorgesehen sind, durch die ein Drehen der Nadel (30) um deren Achse blockierbar ist.

12. Sterile Hülle zu einem Punktiergerät gemäss einem der Ansprüche 1 bis 11.

Fig.1

Fig.2

Fig.3

Fig. 4

Fig. 5a

Fig. 5b

## Fig. 6a

## Fig. 6b

Europäisches
Patentamt

# EUROPÄISCHER RECHERCHENBERICHT

Nummer der Anmeldung

EP  92 81 0313

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.5) |
|---|---|---|---|
| Y<br>A | DE-A-3 329 041 (TOKYO SHIBAURA DENKI K. K.)<br>* Zusammenfassung *<br><br>* Seite 6, Zeile 20 - Seite 7, Zeile 7 *<br>* Seite 9, Zeile 8 - Seite 10, Zeile 5;<br>Abbildungen 3,4,8 *<br><br>--- | 1<br>3,5,7,9,<br>12 | A61B8/08<br>A61B17/34 |
| Y<br>A | WO-A-8 403 034 (H. DRUE)<br>* Seite 2, Zeile 24 - Zeile 32 *<br>* Seite 3, Zeile 27 - Seite 4, Zeile 10;<br>Abbildungen 1-5 *<br><br>--- | 1<br>2,6 | |
| A | EP-A-0 396 874 (ISOTOPEN-TECHNIK DR. SAUERWEIN GMBH)<br>* Spalte 3, Zeile 7 - Zeile 23 *<br>* Spalte 4, Zeile 5 - Zeile 25; Abbildungen *<br><br>----- | 8,9,12 | |
| | | | **RECHERCHIERTE SACHGEBIETE (Int. Cl.5)**<br><br>A61B |

Der vorliegende Recherchenbericht wurde für alle Patentansprüche erstellt

| Recherchenort | Abschlußdatum der Recherche | Prüfer |
|---|---|---|
| DEN HAAG | 02 SEPTEMBER 1992 | FONTENAY P.H. |

EPO FORM 1503 03.82 (P0403)